# EUROPEAN PATENT APPLICATION

(11) **EP 3 811 863 A1**
(43) Date of publication of application: **28.04.2021**
(21) Application number: 20202179.6
(22) Date of filing: 16.10.2020
(51) Int. Cl.: A61B 5/08, A61B 5/29, A61B 5/347

(54) **USING AMPLITUDE MODULATION (AM) OF ELECTROCARDIOGRAM (ECG) SIGNAL RECORDED BY AN IMPLANT TO MONITOR BREATHING**

(30) Priority: 17.10.2019 US 201916655339
(71) Applicant: BIOSENSE WEBSTER (ISRAEL) LTD., 2066717 Yokneam (IL)
(72) Inventor: GOVARI, Assaf, 2066717 Yokneam (IL); FUCHS, Amit, 2066717 Yokneam (IL); ALTMANN, Andres Claudio, 2066717 Yokneam (IL)
(74) Representative: Small, Gary James

(57) **Abstract**

A medical monitoring method includes acquiring, using an implantable heart monitoring device implanted in a patient, an electrocardiogram (ECG) signal that is amplitude modulated (AM) by respiration of the patient. The AM ECG signal is analyzed to identify a respiratory pattern of the patient. The identified respiratory pattern is indicated to a user.

## Description

### FIELD OF THE INVENTION

The present invention relates generally to invasive medical devices, and particularly to methods, apparatuses and software for wireless sensing, e.g., using implanted devices.

### BACKGROUND OF THE INVENTION

Methods for monitoring respiration patterns of patients were previously proposed in the patent literature. For example, U.S. Patent Application Publication 2007/0032733 describes an apparatus for detecting sleep disordered breathing (SDB), cardiac events and/or heart rate variability (HRV) in a subject from an ECG signal. The apparatus includes means for monitoring the ECG signal and means for extracting from the ECG signal parameters indicative of the SDB, cardiac events and/or HRV. The SDB, cardiac events and/or HRV may be detected in real time, breath by breath and/or post acquisition of the ECG signal. A method of detecting SDB, cardiac events and/or HRV in the subject is also disclosed.

As another example, U.S. Patent 6,600,949 describes a method for monitoring the condition of a heart failure patient using respiration patterns. An implantable or other ambulatory monitor senses the patient's respiratory patterns to identify the presence of periodic breathing or Cheyne-Stokes respiration. In an embodiment, heart rate variability (HRV) possibly associated with Cheyne-Stokes respiration are detected. In another embodiment of the invention, modulation of the average heart rate over time is monitored and its absence is used as an indicator of Cheyne-Stokes respiration.

### SUMMARY OF THE INVENTION

An embodiment of the present invention provides a medical monitoring method. The method includes acquiring, using an implantable heart monitoring device implanted in a patient, an electrocardiogram (ECG) signal that is amplitude modulated (AM) by respiration of the patient. The AM ECG signal is analyzed to identify a respiratory pattern of the patient. The identified respiratory pattern is indicated to a user.

In some embodiments, the method further includes alerting the user when the identified respiratory pattern deviates from a normal respiratory pattern according to a prespecified criterion.

In some embodiments, identifying the respiratory pattern includes estimating one or more respiratory rates.

In some embodiments, analyzing the amplitude modulated ECG signal includes spectrally analyzing the amplitude modulated ECG signal.

In an embodiment, identifying the respiratory pattern includes correlating the analyzed AM ECG signal with one or more analyzed AM ECG signals that were each pre-calibrated to indicate a prespecified respiratory pattern.

In another embodiment, the method further includes assigning the identified respiratory pattern with a metric to classify a type of breathing, the metric defined via a degree of correlation between the AM ECG signal and the set of analyzed ECG signals.

In some embodiments, the method further includes verifying the identified respiratory pattern by correlating accelerometer signals from an accelerometer included in the implantable heart monitoring device with one or more accelerometer signals that were pre-calibrated each to indicate a prespecified respiratory pattern.

In some embodiments, the implantable heart monitoring device is an implantable loop recorder (ILR).

In an embodiment, the method further includes determining a position and/or orientation of the implantable heart monitoring device in the patient based on comparing the ECG signal to electrophysiological signals generated by muscular activity of the patient.

In another embodiment, analyzing the AM ECG signal includes performing, in a wireless communication device, an analysis of the received AM ECG signal, and outputting a result of the analysis.

In some embodiments, the method further includes transmitting at least a part of the received AM ECG signal from the wireless communication device over a communication network to a server.

In some embodiments, identifying the respiratory pattern includes identifying a Cheyne-Stokes type of respiratory pattern.

There is additionally provided, in accordance with an embodiment of the present invention, a monitoring apparatus including an implantable heart monitoring device and a processor. The implantable heart monitoring device is configured to be implanted in a patient and to acquire an electrocardiogram (ECG) signal that is amplitude modulated (AM) by respiration of the patient. The processor is configured to analyze the amplitude modulated ECG signal to identify a respiratory pattern of the patient, and indicate the identified respiratory pattern to a user.

The present invention will be more fully understood from the following detailed description of the embodiments thereof, taken together with the drawings in which:

### BRIEF DESCRIPTION OF THE DRAWINGS

Fig. 1 is a schematic pictorial illustration of an apparatus for heart and respiratory monitoring, in accordance with an embodiment of the invention;
Figs. 2A and 2B are graphs that schematically illustrate electrocardiogram (ECG) signals, which are amplitude-modulated by normal and abnormal respiration, respectively, in accordance with embodiments of the invention;
Figs. 3A and 3B are graphs that schematically illustrate frequency components characteristic of the respiratory amplitude-modulated electrocardiogram (AM ECG) signals of Figs. 2A and 2B, respectively, in accordance with embodiments of the invention; and
Fig. 4 is a flow chart that schematically illustrates a method for monitoring breathing of a patient using the cardiac monitoring apparatus of Fig. 1, in accordance with an embodiment of the invention.

### DETAILED DESCRIPTION OF EMBODIMENTS

### OVERVIEW

A patient who requires an implantable heart monitoring device, such as an implantable loop recorder (ILR), for monitoring cardiac events such as arrhythmia, may also require monitoring of breathing. Conventionally, this would entail selecting another specific device (apart of the ILR) and providing the patient with the device and instructions as to how it is to be used to monitor breathing.

Embodiments of the present invention use the fact that different physiological mechanisms related to respiration may cause amplitude modulation (AM) of an ECG signal. These may include changes in venous return and heart stroke volume caused by changing intrathoracic pressure during respiration, changes in tissue volume due to varying arterial pressure, and respiratory sinus arrhythmia, for example. Electrophysiologically, the above listed physiological mechanisms may cause an amplitude modulation of the ECG signal due to the respiration changing an orientation of the electrical axis of the heart relative to the electrodes of a monitoring device such as the ILR, and/or by changing thoracic impedance.

Embodiments of the present invention that are described hereinafter use an apparatus comprising an implantable heart monitoring device (e.g., the ILR) to also monitor patient breathing. A processor used by the apparatus analyzes a spectrum of respiratory amplitude-modulated electrocardiogram (AM ECG) signal acquired by the ILR. The analyzed spectrum of the AM ECG signal includes frequency components which are indicative of various possible normal and abnormal respiratory patterns, and therefore can be used to monitor breathing.

In some embodiments, a medical monitoring method is provided, which includes: (a) acquiring, using the implantable heart monitoring device, an ECG signal that is amplitude-modulated by respiration, (b) analyzing the amplitude-modulated ECG signal to identify a respiratory pattern, and (c) indicating the identified respiratory pattern to a user, including alerting the user when the identified respiratory pattern deviates from a normal respiratory pattern according to prespecified criteria (e.g., by comparison against an AM ECG signal database of normal and abnormal respiratory patterns).

In some embodiments, the disclosed method includes estimating one or more respiratory rates. In other embodiments, the ILR is used to monitor apnea, and to correlate breathing, apnea, and heart rate. As shown below, apnea may involve the appearance of multiple distinctive respiratory rates (i.e., of frequency components) in the analyzed AM ECG spectrum. In some embodiments, the processor spectrally analyzes the AM ECG signal by applying Fourier analysis.

The amplitude-modulation of the ECG signals can be analyzed by a local processor and/or a remote processor. In some embodiments, the processor identifies a respiratory pattern by correlating the analyzed amplitude-modulated ECG signal with one or more analyzed ECG signals that were each pre-calibrated to indicate prespecified respiratory patterns.

In some embodiments, the processor correlates a spectrum of the amplitude-modulated ECG signal with a given set of spectra of respiratory patterns that were pre-calibrated to indicate prespecified respiratory patterns. In an embodiment, the given set of spectrally analyzed normal and abnormal breathing patterns comprises the patient's own respiratory patterns. A correlation of the analyzed ECG signals can be used to provide a metric for the breathing, and to systemize a classification of a type of observed breathing. The metric may be defined via a degree of the above correlation to any spectrum of the pre-calibrated respiratory patterns or to a known spectrum.

Typically, the ECG signal has background electrophysiological signals, such as those generated by muscular activity, known as muscular disturbances. Electrophysiological signals from muscle disturbances may peak, for example, when chest muscles contract. The spectrum of such an electrophysiological interference may overlap the spectrum of the cardiac ECG signals and thereby cause electrophysiological noise. In some embodiments, the position and/or orientation of the ILR implant in the body is optimized to obtain a clear ECG signal against background electrophysiological signals (e.g., to increase the signal to noise ratio). For example, by carefully positioning and/or orienting the ILR, the quality of raw ECG data is improved, leading to better monitoring capabilities.

In an optional embodiment, an accelerometer is added to the ILR to detect patient motion, such as chest wall motion. A processor using accelerometer signals may verify an ECG-identified respiratory pattern by correlating the accelerometer signals with one or more pre-calibrated accelerometer signals that indicate particular respiratory patterns.

Typically, the local and/or remote processors are programmed in software containing a particular algorithm that enables the processors to conduct each of the processor-related steps and functions outlined above.

By enabling dual usage of an ILR implant for cardiac and respiratory monitoring, embodiments of the present invention save the patient and the healthcare provider the medical complexity, effort, and costs involved of using two independent and unrelated monitoring devices.

### SYSTEM DESCRIPTION

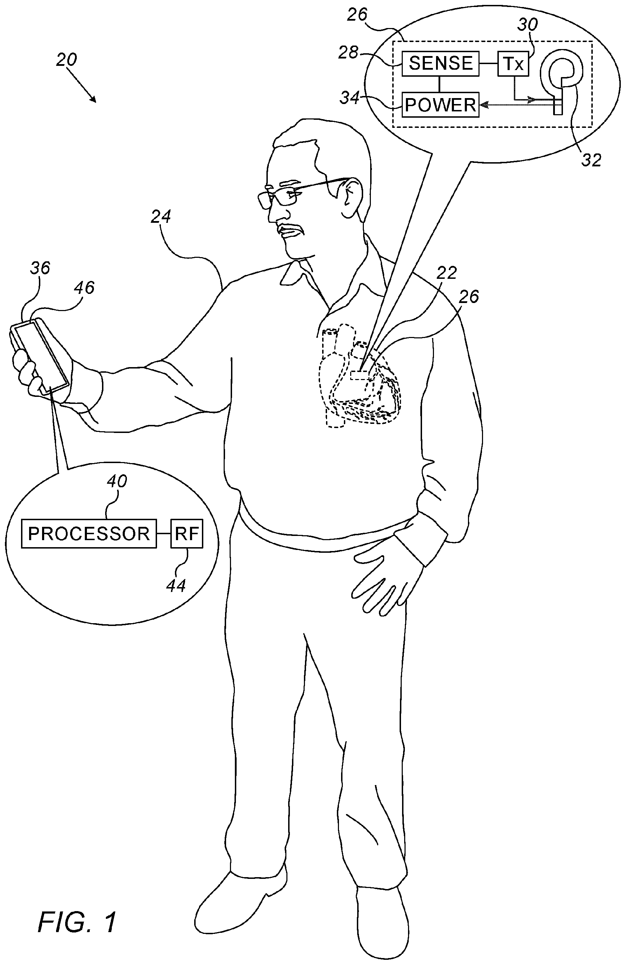

Fig. 1 is a schematic pictorial illustration of an apparatus 20 for heart and respiratory monitoring, in accordance with an embodiment of the invention. Apparatus 20 is built around an implantable heart monitoring device 26, in the present example an ILR. Cardiac monitoring apparatuses comprising implantable heart monitoring devices, such as device 26, are described in U.S. Patent 10,165,125, assigned to the assignee of the present patent application, and whose disclosure is incorporated herein by reference.

In the present example, device 26 is implanted, typically via an incision or injection through the skin, in the chest of a patient 24 in proximity to a heart 22 that is to be monitored. Device 26 comprises a sensor 28, typically comprising an electrode or electrodes, which senses and records physiological activity, such as electrical signals generated by heart 22. When actuated, a transmitter 30 transmits recorded signal data via an integral antenna 32, represented in the figure as a coil. A power source 34, such as a battery and/or chargeable capacitor, supplies operating power to sensor 28 and transmitter 30, and may be rechargeable by means of radiofrequency (RF) energy received via antenna 32.

Patient 24 operates a smartphone 36 to receive and analyze the data transmitted by transmitter 30. Smartphone 36 is a standard, off-the-shelf device with mobile telephony, sensing, and processing capabilities. For the sake of brevity, only those elements of smartphone 36 that are directly relevant to interaction with implantable device 26 are described here. In the pictured example, smartphone 36 comprises a processor 40, along with a short-range RF transceiver 44, such as a Bluetooth® or RF identification (RFID) transceiver. Smartphone 36 also comprises a user interface 46, comprising a touchscreen, as well as audio input and output.

Processor 40 carries out the functions that are described herein under the control of software, which is stored in a tangible, non-transitory memory (not shown), such as semiconductor, optical or magnetic memory. Typically, the software is in the form of an application program, which is downloaded to smartphone 36 in electronic form, over a network, although the software may alternatively be pre-installed in the smartphone or supplied on tangible media. After installation, patient 24 or another user launches the application so that processor 40 will be ready to collect data from implanted device 26 on demand.

Upon receiving the data (e.g., ECG data), processor 40 may perform an analysis of the received information, and output the result. For example, processor 40 may output a graphical image and/or sound via user interface 46 to inform patient 24 that cardiac activity is normal, or alternatively that a possible arrhythmia has been detected and that the patient should seek medical care. At the same time, processor 40 may output a graphical image and/or sound via user interface 46 to inform patient 24 that respiratory activity is normal, or alternatively that a possible respiratory condition has been detected and that the patient should seek medical care.

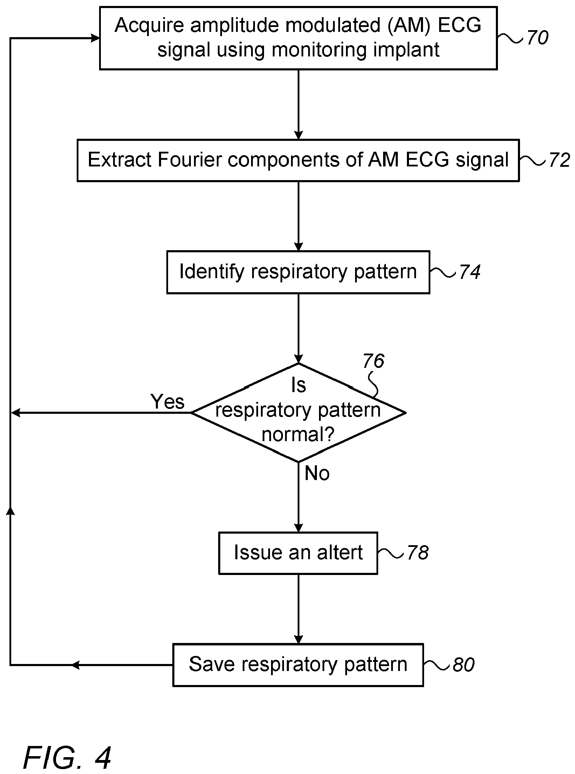

In particular, processor 40 runs a dedicated algorithm as disclosed herein, included in Fig. 4, that enables processor 40 to perform the disclosed steps, as further described below.

Additionally or alternatively, processor 40 may output at least part of the received data and/or results of analysis via a communication network (such as a cellular or Wi-Fi data network) to a server.

As another option, in embodiments in which power source 34 is rechargeable, transceiver 44 may also transmit RF energy to antenna 32 in order to charge the power source when the wireless communication link is actuated. Power source 34 rectifies and stores the energy in order to drive sensor 28 and transmitter 30. Thus, the useful lifetime of device 26 inside the body may be extended by recharging power source 34 every time transmitter 30 is interrogated.

### USING AM ECG SIGNAL RECORDED BY AN IMPLANT TO MONITOR BREATHING

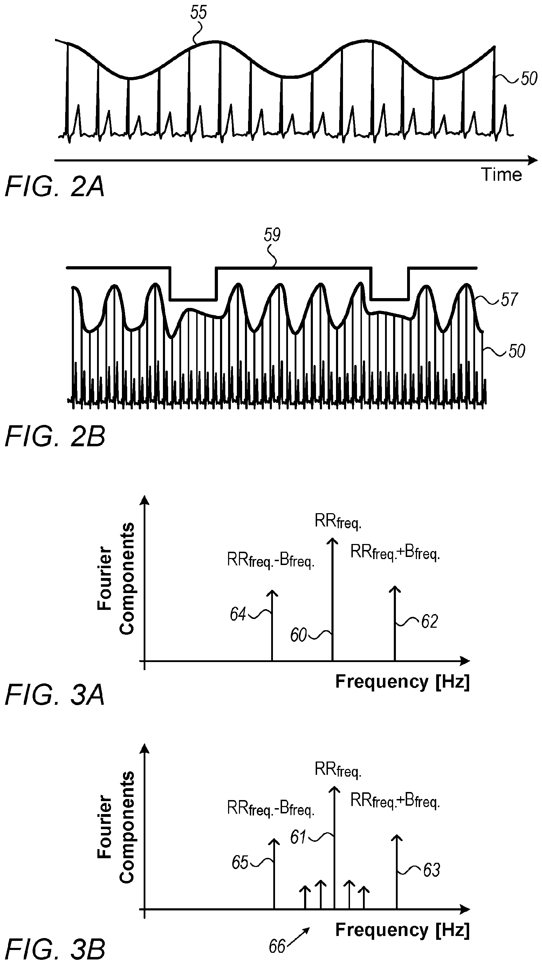

Figs. 2A and 2B are graphs that schematically illustrate electrocardiogram (ECG) signals 50, which are amplitude-modulated by normal and abnormal respiration, respectively, in accordance with embodiments of the invention. As seen, a higher rate periodic ECG waveform (50) having a characteristic heartbeat rate is slowly amplitude-modulated by a respiratory signal (55, 59). In Fig. 2A, the resulting AM ECG signal shows near-sinus rhythm amplitude modulation (55) of ECG signal 50, which is indicative of a normal breathing pattern. This visual impression is quantified by the spectral analysis shown in Fig. 3A.

In Fig. 2A, the rate of breathing is about a fifth of that of the heart rate. In general, the rate of normal breathing and normal heart rate may be time-dependent (e.g., drift and/or vary), depending, for example, on physical conditions.

In some cases, breathing and the heart rates may vary or fluctuate with medical condition, which, for example, causes respiratory and heart rates to have complex reoccurring patterns, or to display unstable rates.

Fig. 2B shows a rectangular amplitude modulation (57) of an ECG signal, which otherwise exhibits normal sinus modulation. Such an AM ECG signal is characteristic of abnormal breathing caused by sleep apnea. Specifically, the abnormal breathing is a Cheyne-Stokes type of respiratory pattern, as deduced from the spectral analysis shown in Fig. 3B. The long pauses in breathing are manifested in Fig. 2B as a slow rectangular modulation (59) of the normally amplitude-modulated (57) ECG signal 50.

For example, while a normal breathing rate is about 15 Hz, the repeated intermissions in breathing has a rate of few Hertz, as schematically shown in Fig. 2B by the rectangular modulation.

Figs. 3A and 3B are graphs that schematically illustrate frequency components characteristic of the respiratory amplitude-modulated electrocardiogram (AM ECG) signals of Figs. 2A and 2B, respectively, in accordance with embodiments of the invention. For the sake of simplicity, only few dominant spectral (e.g., Fourier) components are schematically shown, whereas an actual spectrum may include multiple peaks, with each peak being broadened by various inhomogeneities, such as those caused by, for example, unrelated biophysical activity.

As seen in Fig. 3A, the heartbeat is characterized by a carrier-frequency rate 60, *RR_{freq.}.* The Fourier spectrum of the AM ECG signal shows symmetrical satellite peaks 62 and 64 of main peak 60, with the two satellite peaks found at the two frequencies *RR_{freq.}*+*B_{freq.}* and *RR_{freq.}*-*B_{freq.}.* The shown spectrum thus shows sinusoidal AM, having a modulation rate *B_{freq.},* which indicates normal respiration.

In Fig. 3B, the heartbeat is also characterized by a carrier-frequency rate 61, *RR_{freq.}.* The Fourier spectrum of the AM ECG signal still includes symmetrical satellite peaks 61 and 63, i.e., sinusoidal components generated due to a normal sinus modulation rate *B_{freq}.* However, the Fourier transformed respiration-induced AM pattern additionally includes a host of closely packed satellite peaks 66.

To interpret peaks 66, Fig. 2B above is revisited, which shows how sleep apnea modulates the breathing itself, very slowly, by causing breathing intermissions. These intermissions have a distinctive spectral signature comprising satellite peaks 66. When processor 40 identifies the presence of satellite peaks 66, such as shown schematically in Fig. 3B, the processor alerts the user of abnormal breathing and saves the abnormal respiratory pattern for further examination.

In general, a respiratory pattern does not have a pure sinus rhythm; instead, a respiratory pattern may comprise one or more respiratory rates. Therefore, simple spectral analysis as shown in Fig. 3 may not be sensitive and specific enough. In some embodiments, to differentiate one respiratory pattern from another, a processor may, as described above, correlate the spectrally analyzed amplitude-modulated ECG signal with a given set of spectrally analyzed AM ECG signals that were pre-calibrated using a respective set comprising normal and abnormal prespecified respiratory patterns, so as to indicate respiratory patterns.

In an embodiment, the processor may correlate a spectral signature such as shown in Fig. 3B with a given set of spectral signatures that were generated by spectrally analyzing a set of (e.g., one or more) AM ECG signals acquired during normal and abnormal respiration of the patient.

Figs. 3A and 3B are brought by way of example. As another example of a distinct signature, a high frequency satellite that is indicative of hyperventilation may occur. As yet another example, multiple frequency components of breathing, including both low and high amplitudes, may indicate of an obstacle in breathing, which can occur in a Biot's type of respiration.

Fig. 4 is a flow chart that schematically illustrates a method to monitor breathing of a patient using the cardiac monitoring apparatus 20 of Fig. 1, in accordance with an embodiment of the invention. The algorithm according to the presented embodiment carries out a process that begins with implantable heart monitoring device 26 acquiring an AM ECG signal, which is typically amplitude-modulated, at an AM ECG acquisition step 70. Next, processor 40 analyzes the ECG signal and extracts, for example using Fourier transform techniques, frequency components in the spectrum of the AM ECG signal generated by the amplitude-modulation, at an AM components extraction step 72.

Next, based on the spectral analysis of the amplitude-modulated ECG signal, processor 40 identifies a respiratory pattern comprising one or more respiratory rates, at a respiratory pattern identification step 74.

At a checking step 76, processor 40 checks the respiratory pattern it identified against a database of respiratory patterns, for example by correlating the extracted spectrum with a set of pre-calibrated spectra of respiratory patterns. If, based on the correlation, processor 40 concludes that the identified respiratory pattern is abnormal, processor 40 issues an alert, at an alerting step 78. Additionally, processor 40 stores the abnormal findings is a memory of apparatus 20, at a saving data step 80. Finally, whether or not the identified respiratory pattern is found to be abnormal, the process returns to step 70 to acquire new ECG signals.

The present embodiment also comprises additional steps of the algorithm, such as analyzing heart rhythm, which have been omitted from the disclosure herein purposely on order to provide a more simplified flow chart.

Although the embodiments described herein mainly address the use of invasive devices, the methods and apparatuses described herein can also be used, *mutatis mutandis,* with non-invasive devices, for applications such as those used in sports.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A medical monitoring method, the method comprising:
acquiring, using an implantable heart monitoring device implanted in a patient, an electrocardiogram (ECG) signal that is amplitude modulated (AM) by respiration of the patient;
analyzing the AM ECG signal to identify a respiratory pattern of the patient; and
indicating the identified respiratory pattern to a user.

2. The method according to claim 1, and comprising alerting the user when the identified respiratory pattern deviates from a normal respiratory pattern according to a prespecified criterion.

3. The method according to claim 1, wherein identifying the respiratory pattern comprises estimating one or more respiratory rates.

4. The method according to claim 1, wherein analyzing the amplitude modulated ECG signal comprises spectrally analyzing the amplitude modulated ECG signal.

5. The method according to claim 1, wherein identifying the respiratory pattern comprises correlating the analyzed AM ECG signal with one or more analyzed AM ECG signals that were each pre-calibrated to indicate a prespecified respiratory pattern.

6. The method according to claim 5, and comprising assigning the identified respiratory pattern with a metric to classify a type of breathing, the metric defined via a degree of correlation between the AM ECG signal and the set of analyzed ECG signals.

7. The method according to claim 1, and comprising verifying the identified respiratory pattern by correlating accelerometer signals from an accelerometer included in the implantable heart monitoring device with one or more accelerometer signals that were pre-calibrated each to indicate a prespecified respiratory pattern.

8. The method according to claim 1, wherein analyzing the AM ECG signal comprises performing, in a wireless communication device, an analysis of the received AM ECG signal, and outputting a result of the analysis.

9. The method according to claim 8, and comprising transmitting at least a part of the received AM ECG signal from the wireless communication device over a communication network to a server.

10. The method according to claim 1, wherein identifying the respiratory pattern comprises identifying a Cheyne-Stokes type of respiratory pattern.

11. A monitoring apparatus, comprising:
an implantable heart monitoring device, which is configured to be implanted in a patient and to acquire an electrocardiogram (ECG) signal that is amplitude modulated (AM) by respiration of the patient; and
a processor, which is configured to:
analyze the amplitude modulated ECG signal to identify a respiratory pattern of the patient; and
indicate the identified respiratory pattern to a user.

12. The monitoring apparatus according to claim 11, wherein the processor is further configured to alert the user when the identified respiratory pattern deviates from a normal respiratory pattern according to a prespecified criterion.

13. The monitoring apparatus according to claim 11, wherein the processor is configured to identify the respiratory pattern by estimating one or more respiratory rates.

14. The monitoring apparatus according to claim 11, wherein the processor is configured to analyze the amplitude modulated ECG signal by spectrally analyzing the amplitude modulated ECG signal.

15. The monitoring apparatus according to claim 11, wherein the processor is configured to identify the respiratory pattern by correlating the analyzed AM ECG signal with of the patient analyzed AM ECG signals that were each pre-calibrated to indicate a prespecified respiratory pattern.

16. The monitoring apparatus according to claim 15, wherein the processor is further configured to assign the identified respiratory pattern with a metric to classify a type breathing, the metric defined via a degree of correlation between the AM ECG signal and the set of analyzed ECG signals.

17. The monitoring apparatus according to claim 11, wherein the processor is further configured to verify the identified respiratory pattern by correlating accelerometer signals from an accelerometer included in the implantable heart monitoring device with one or more accelerometer signals that were pre-calibrated each to indicate a prespecified respiratory pattern.

18. The method according to claim 1 or the monitoring apparatus according to claim 11, wherein the implantable heart monitoring device is an implantable loop recorder (ILR) .

19. The method according to claim 1 or the monitoring apparatus according to claim 11, and comprising determining a position and/or orientation the implantable heart monitoring device in the patient based on comparing the ECG signal to electrophysiological signals generated by muscular activity of the patient.

20. The monitoring apparatus according to claim 11, and comprising a wireless communication device that is configured to perform an analysis of the received AM ECG signal, and output a result of the analysis.

21. The monitoring apparatus according to claim 20, wherein the wireless communication device is configured to transmit at least a part of the received AM ECG signal over a communication network to a server.

22. The monitoring apparatus according to claim 11, wherein the processor is configured to identify the respiratory pattern by identifying a Cheyne-Stokes type of respiratory pattern.
